(19) Europäisches Patentamt
European Patent Office
Office européen des brevets

(11) EP 2 570 413 A1

(12) **EUROPEAN PATENT APPLICATION**

(43) Date of publication:
**20.03.2013 Bulletin 2013/12**

(51) Int Cl.:
***C07D 417/04*** *(2006.01)* ***A61K 31/5415*** *(2006.01)*
***A61P 31/06*** *(2006.01)*

(21) Application number: **11181388.7**

(22) Date of filing: **15.09.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **The University Of Queensland
St. Lucia, Queensland 4072 (AU)**

(72) Inventors:
- **Cooper, Matthew
Chapel Hill, Queensland 4069 (AU)**
- **Zuegg, Johannes
Wynnum, Queensland 4178 (AU)**
- **Becker, Bernd
New Farm, Queensland (AU)**
- **Karoli, Tomislav
Kenmore, Queensland 4069 (AU)**

(74) Representative: **HOFFMANN EITLE
Patent- und Rechtsanwälte
Arabellastrasse 4
81925 München (DE)**

(54) **Benzothiazinone derivatives as anti-tuberculosis agents**

(57) Novel benzothiazinone derivatives of formula (I) or pharmaceutically acceptable salts or solvates thereof have been found to be effective against *Mycobacterium tuberculosis* strains and may thus be useful in the treatment of tuberculosis:

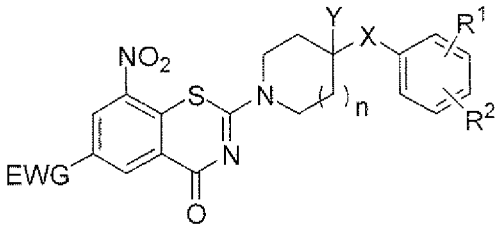

(I)

wherein
EWG (electron withdrawing group) = $NO_2$ CN, $CF_3$, F, Cl, Br, $OCF_3$, OH, OR, $OCHF_2$, COOR, wherein R is hydrogen or a straight or branched $C_1$-$C_4$ alkyl group,
X = a bond or a straight or branched $C_1$-$C_4$ alkylene group which may be substituted with a group selected from F, Cl, Br, I or $C_1$-$C_4$ alkoxy;
Y = hydrogen, a straight or branched $C_1$-$C_4$ alkyl group, OH or OR, wherein R is hydrogen or a straight or branched $C_1$-$C_4$ alkyl group;
n = 0, 1 or 2;
$R^1$, $R^2$ = hydrogen or substituent(s) which may be the same or different from each other and are selected from the group consisting of D, F, Cl, Br, $CF^3$, a straight or branched $C_1$-$C_4$ alkyl group, a phenyl group, OR, SR, $NR^3R^4$, wherein R, $R^3$, $R^4$ may be the same or different from each other and are hydrogen or a straight or branched $C_1$-$C_4$ alkyl group or a phenyl group.

EP 2 570 413 A1

## Description

### Field of the Invention

The present invention relates to novel benzothiazinone derivatives of formula (I) that kill *Mycobacterium tuberculoses* by blocking Arabian Synthesis and is further directed to a process for the preparation of the compounds of formula (I).

### Background of the Invention

The loss of human lives to tuberculosis (TB) continues essentially unabated as a result of poverty, synergy with the HIV/AIDS pandemic and the emergence of multidrug resistant (MDR) and extensively drug resistant (XDR) strains of *Mycobacterium tuberculosis.* It is therefore necessary to continually provide innovations in this field to combat these highly dangerous infections, particularly when they are caused by strains that are resistant to the existing anti TB drugs, like rifampicin, isonazid, pyrazinamid, ethambutol as first line TB drugs or further second line TB drugs, as e.g. chinolones and aminoglycosides.

EP 2 176 248 B1 (WO 2009/010163) describes the use of certain piperazine-substituted benzothiazinones as effective agents for the treatment and prevention of tuberculosis and leprosy caused by *Mycobacteria.* It was found that the novel compounds according to the present invention have at least the same antimicrobial efficacy as the compounds of EP 2 176 248 B1, and in addition a superior profile of pharmacologically important properties such as stability, solubility and toxicity.

WO 2007/134625 and EP 2 020 406 describe further antimicrobial compounds of the benzothiazinone class and their use as antibacterial agents in infectious diseases of mammals (humans and animals) caused by bacteria, especially *Mycobacteria.*

### Summary of the invention

The present invention provides novel benzothiazinone derivatives of formula (I) (see below), which are biologically active and which were found to be effective against *Mycobacteria* strains that are known to cause tuberculosis and leprosy. In addition, the compounds of the present invention have a very good and generally improved profile of pharmacologically relevant properties such as stability and toxicity over known compounds of the same class. The present compounds are suitable for the prevention and treatment of tuberculosis and leprosy caused by the multidrug- and extensively drug resistant strains of *Mycobacterium tuberculosis.*

### Disclosure of the invention

In a first embodiment, the compounds of the present invention are represented by compounds of formula (I) or pharmacologically acceptable salts or solvates thereof as shown in the following:

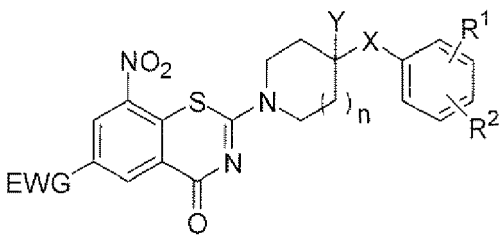

(I)

wherein

EWG (electron withdrawing group) = $NO_2$, CN, $CF_3$, F, Cl, Br, $OCF_3$, OH, OR, $OCHF_2$, COOR, wherein R is hydrogen or a straight or branched $C_1$-$C_4$ alkyl group,

X = a bond or a straight or branched $C_1$-$C_4$ alkylene group which may be substituted with a group selected from F, Cl, Br, I or $C_1$-$C_4$ alkoxy;

Y = hydrogen, a straight or branched $C_1$-$C_4$ alkyl group, OH or OR, wherein R is hydrogen or a straight or branched $C_1$-$C_4$ alkyl group;

n = 0, 1 or 2;

$R^1$, $R^2$ = hydrogen or substituent(s) which may be the same or different from each other and are selected from

the group consisting of D, F, Cl, Br, $CF_3$, a straight or branched $C_1$-$C_4$ alkyl group, a phenyl group, OR, SR, $NR^3R^4$, wherein R, $R^3$, $R^4$ may be the same or different from each other and are hydrogen or a straight or branched $C_1$-$C_4$ alkyl group or a phenyl group.

In a second embodiment, the present invention is directed to a compound of formula (I) or pharmacologically acceptable salt or solvate thereof, wherein

$$EWG = CF_3;$$

X = a bond or a straight or branched $C_1$-$C_4$ alkylene group which may be substituted with a group selected from F, C1, Br, I, or $C_1$-$C_4$ alkoxy;
Y = hydrogen, a straight or branched $C_1$-$C_4$ alkyl group, OH or OR, wherein R is hydrogen or a straight or branched $C_1$-$C_4$ alkyl group;
n = 1;
$R^1$, $R^2$ = hydrogen or substituent(s) which may be the same or different from each other and are selected from the group consisting of D, F, Cl, Br, $CF_3$, a straight or branched $C_1$-$C_4$ alkyl group, a phenyl group, OR, SR, $NR^3R^4$, wherein R, $R^3$, $R^4$ may be the same or different from each other and are hydrogen, or a straight or branched $C_1$-$C_4$ alkyl group or a phenyl group.

In a third embodiment, the present invention relates to compounds of formula (I) or pharmacologically acceptable salts or solvates thereof, wherein
EWG = $CF_3$;
X = a bond or $-CH_2-$ group;
Y = hydrogen or hydroxy;
n = 1;
$R^1$, $R^2$ = hydrogen or substituent(s) which may be the same or different from each other and are selected from the group consisting of D, F, Cl, Br, $CF_3$, a straight or branched $C_1$-$C_4$ alkyl group, a phenyl group, OR, SR, $NR^3R^4$, wherein R, $R^3$, $R^4$ may be the same or different from each other and are hydrogen or a straight or branched $C_1$-$C_4$ alkyl group or a phenyl group.

Particularly preferred compounds of the invention are those according to any of the first, second or third embodiments (the same applies for the following preferred compounds), wherein X is a bond.

Further preferred compounds of the invention include those according to any of the first, second or third embodiments, wherein Y is a hydrogen or hydroxyl group or wherein $R^1$ is hydrogen, F, Cl or a $CH_3$-group and $R^2$ is hydrogen or F.

The most preferred compounds according to formula (I) are selected from the group consisting of:

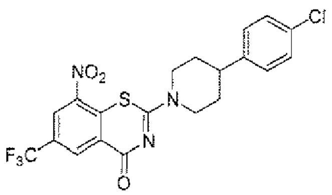
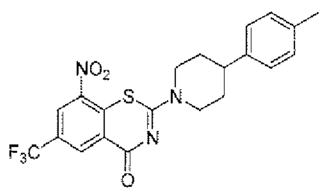
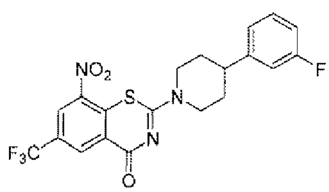
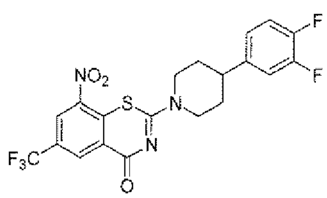
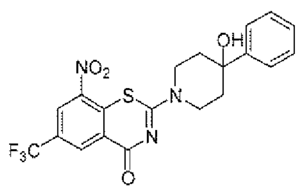
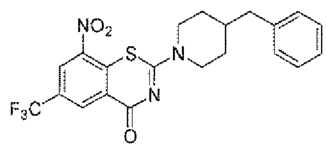

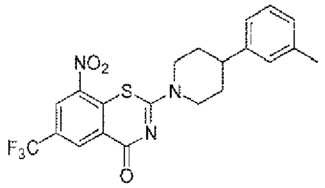 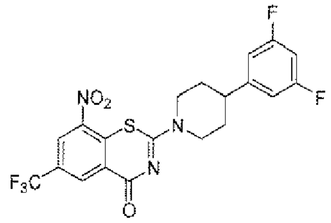 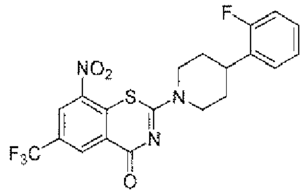

and

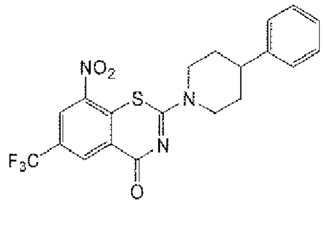.

Presently particularly preferred compounds are the following:

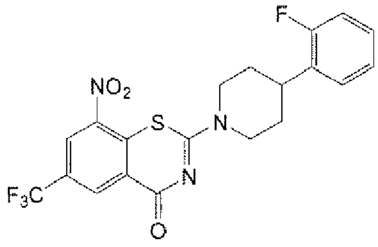 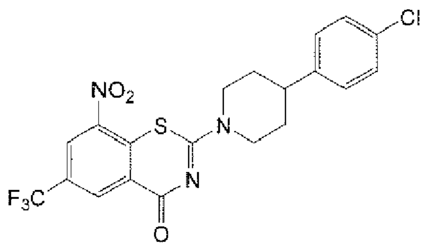

Likewise, pharmaceutically acceptable salts or solvates of either of the above compounds are preferred which may help to improve both the solubility and the bioavailability of the free compounds described above.

The compounds of the present invention were found to be effective as antimicrobial agents, particularly against various strains of *Mycobacteria* and thus, are potential candidates for inclusion in single or combination therapies for both drug-sensitive and extensively drug resistant TB. The compounds of formula (I) or their pharmaceutically acceptable salts or solvates are therefore useful in therapy and in a method for treating or preventing of a microbial infection in mammals, particularly humans. In particular, the compounds of formula (I) or their pharmaceutically acceptable salts or solvates are preferably useful in a method for treating or preventing of a microbial infection, wherein the microbial infection is a tuberculosis or leprosy infection.

The present invention therefore also provides a pharmaceutical composition, particularly a tuberculosis medicament, containing a compound of formula (I) as shown above as the active ingredient, optionally together with one or more pharmaceutically acceptable excipients and/or other therapeutic agents. In addition, such a composition may contain other antimicrobial compounds for which an activity against *Mycobacteria* has been demonstrated.

The invention is further directed to a process for the preparation of compounds of formula (I) comprising the reaction of an S-methyl benzothiazinone derivative according to formula (II) with an amine according to formula (III) or a salt thereof, wherein R is H or trimethylsilyl and all other variable residues are as defined in formula (I), to form a compound of formula (I).

Such a process is shown in Scheme 1 below;

Scheme 1: Reaction of thiomethyl intermediate of formula (II) with an amine of formula (III)

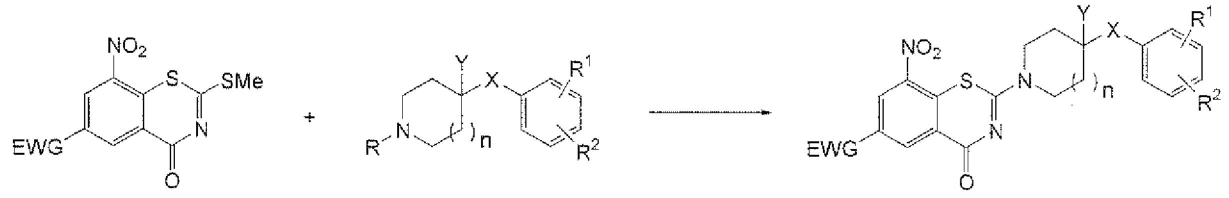

(II) (III) (I)

In this reaction, a nucleophilic addition of an amine of formula (III) to the thiomethyl benzothiazinone core (II) is performed. This synthetic route allows for a very convenient point of convergence in the synthesis as the benzothiazinone core can be prepared on large scale and any number of amines can then be added. The reaction of (II) with (III) is conveniently carried out in a suitable organic solvent, e.g. a lower alcohol having 1-4 carbon atoms, most preferably ethanol, or in another polar organic solvent, most preferably tetrahydrofuran (THF). The reaction is conveniently carried out under slight heating, e.g. at 40-80°C, preferably at 55-65°C.

The synthesis of (II) can be performed in four steps beginning e.g. from 4-chlorobenzotrifluoride. 4-Chlorobenzotrifluoride is carboxylated by selective lithiation and trapping with carbondioxide. Nitration, conversion to the amide *via* the acid chloride, thiazole ring closure using carbondisulfide and methylation yields the thiomethyl key intermediate (II). This intermediate is then reacted directly with the desired amine (III) or a salt thereof to give the compounds of formula (I). The entire process is summarized in Scheme 2.

Scheme 2: Synthesis of benzothiazinones according to the present invention

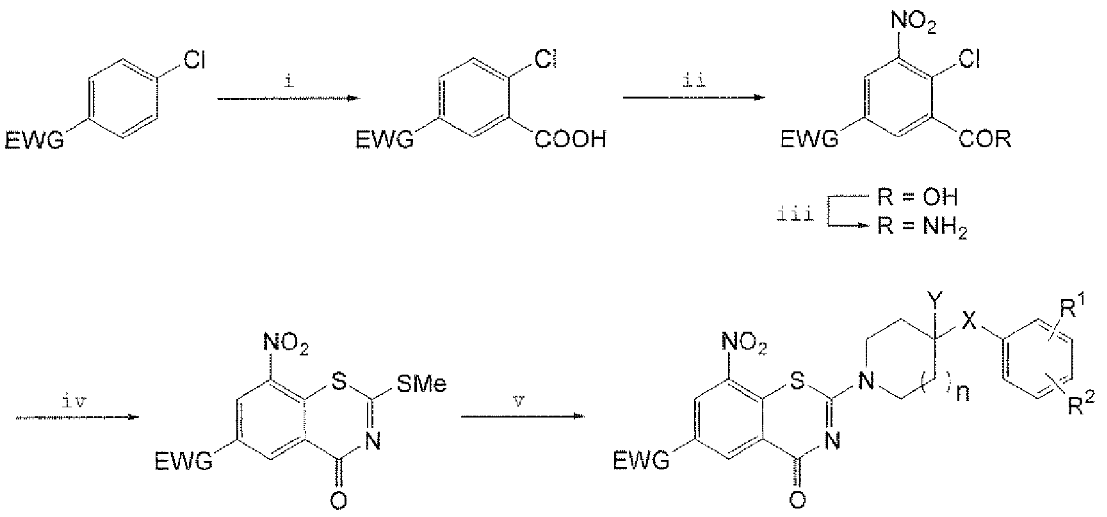


(II) (I)

Suitable reaction conditions for each of the above steps are shown below:

i) n-BuLi, TMEDA then $CO_2$; ii) $H_2SO_4$, $HNO_3$; iii) $SOCl_2$, DMF then $NH_3$; iv) NaOH, $CS_2$, $CH_3I$, DMSO; V) amine of formula (III) - see below -, EtOH, 60°C.

Subsequently, the compound of formula (I) may be converted into one of its pharmaceutically acceptable salts, particularly a pharmaceutically acceptable acid addition salt thereof, by reacting the compound with a pharmaceutically

acceptable acid such as hydrochloric acid, citric acid or the like.

Alternatively, compounds of formula (I) may be converted into one of its pharmaceutically acceptable solvates by addition of solvent molecules to form, for example, a hydrate, an alcoholate or other solvates formed by addition of, for example, a polar solvent, an ester solvent, a ketone solvent or the like.

The obtained compound may further be purified by conventional methods such as crystallization, chromatography or the like.

Further processes for synthesizing the compounds of formula (I) can be taken from the aforementioned WO 2009/010163 and WO 2007/134625 *mutatis mutandis.* Starting materials or useful intermediates can be well known polysubstituted 2-chloro(bromo)-benzcarboxamides, many of which are described in the literature or can be easily prepared by analogous methods (Isaew S. G. Farm. Zh., (Kiev) 2000, 52; Makosza M., Nizamov, S. Org. Prep. and Proced. Int. 1997, 29, 707; Nerin C., Tornes A. R., Domento C., Cacho J., J. Agr. and Food Chem. 1996, 44, 4009; Thiel W., Mayer R., Jauer E.-A., Modrow H., Dost H., J. Prakt. Chem. 1986, 328, 497; Yokoyama M., Yoshida S., Imamoto T., Synthesis 1982, 591; Romanowski J., Eckstein Z. Pol. J. Chem. 1984, 58, 263; Nisato D., Sacilotto R., Frigerio M., Boveri S., Palmisano G., Lesma G., Org. Prep. Proced. Int. 1985, 17, 75; Oikawa N., Nakagawa Y., Nishimura K., Ueno T., Fujita T., Pestle, Sci. 1994, 41, 139; Welch D.E., Baron R.R., J. Med. Chem. 1969, 12, 299; Fuller R.W., Molloy B.B., Day W.A., Roush B.W., March M.M., J. Med. Chem. 1973, 16, 101 and many others).

Pharmaceutically acceptable salts or solvates of compounds of formula (I) form a further aspect of the present invention. They particularly include both acid addition salts such as the hydrochloride, hydrobromide or toluene sulfonate and hydrate or alcoholates, and the like.

The compounds of the present invention can be used in medicaments, particularly oral or parenteral medicaments such as tablets, capsules, injection or infusion solutions. Optionally, and in some cases preferably, such medicaments also contain one or two further antibacterial agents that have activity against *Mycobacteria*, such as TMC 207 or other diaryl quinolines, bicyclic nitroimidazole compounds, isoniazid, rifampicin or pyrazinamide. Such combination medicaments form a further aspect of the present invention. The compounds can be used in dosages from 0.001 - 1000 mg/kg body weight; the exact dosage will be determined by the attending physician and will also depend on the particular compound used.

A method for treating a *Mycobacterium* infection by administering to a patient in need thereof a therapeutically effective dose of a compound of formula (I) or a pharmaceutically acceptable salt or solvate thereof is a further aspect of the present invention.

Likewise, the present invention further envisages a compound of formula (I) or a pharmaceutically acceptable salt or solvate thereof for use in therapy, particularly in the treatment of infections and most particularly in the treatment of infections caused by *Mycobacteria* such as tuberculosis.

**Synthesis Examples**

In the following the synthesis of exemplary compounds of formula (I) is described in more detail.

<u>Preparation of 2-chloro-5-trifluoromethylbenzoic acid</u>

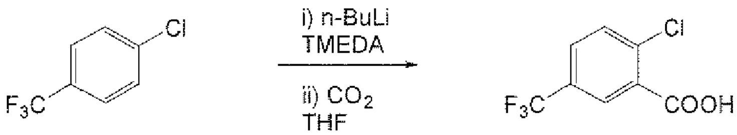


*n*-BuLi (2.5M in hexanes, 22 mL, 55 mmol) was added over 8 minutes to a stirred solution of 4-chlorobenzotrifluoride (9.177 g, 50.8 mmol) and TMEDA (6.3030 g, 54.2 mmol) in THF (89 mL) at -78°C under nitrogen. After 52 min the solution was transferred via cannula into dry ice (~200g) over 20 min. Care was required to sufficiently lag the cannula as the anion solution was found to darken rapidly on slight warming. The mixture was allowed to warm to ambient temperature with stirring and was evaporated (<30°C) to give an orange/yellow solid. The solid was dissolved in water (70 mL) and washed with diethyl ether (3×30 mL). The aqueous layer was acidified to pH 1 and extracted with DCM (3×30 mL). The organic layer was evaporated to dryness (< 30°C) and the residue was dissolved in refluxing hexanes, hot filtered and crystallized by cooling to 4°C overnight. The sandy coloured solid was isolated by vacuum filtration (4.5243 g). The mother liquors were concentrated by half and a second crop of lemon yellow crystals was obtained (2.2998 g). The products were dried under vacuum to give 4.5030 g and 2.2093 g respectively, giving a combined yield of 6.712 g (59 %). LCMS: $R_t$ = 5.94 min, first crop = 96.3 A% @ 254 nm, second crop 95.1 A%, $[M+H]^+$ 225.0, with no

starting material detected.

Preparation of 2-chloro-3-nitro-5-trifluoromethylbenzoic acid

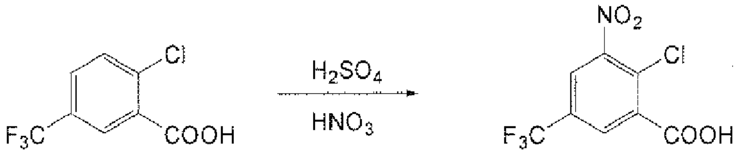


2-Chloro-5-trifluoromethylbenzoic acid (6.5044 g, 28.965 mmol) was added to a solution of sulfuric acid (23 mL) and fuming nitric acid (3.2 mL), with an overhead stirring. The mixture was warmed to 90°C over 26 min, then a further 10 mL of sulfuric acid was added to the thick mixture and stirring was continued. After 45 min at 90°C the mixture was cooled to ambient temperature and poured into crushed ice (150 g), rinsing forward with icy water (50 mL). The product was isolated by vacuum filtration, washed with cold water, and dried *in vacuo* to give 7.3752 g (94.7 %). LCMS: $R_t$ = 5.74 min, 100 A% @ 254 nm, $[M-H]^-$ = 268.0. Co-injection of the product and starting material revealed the compounds were baseline separated.

Preparation of 2-Chloro-3-nitro-5-(trifluoromethyl)benzamide

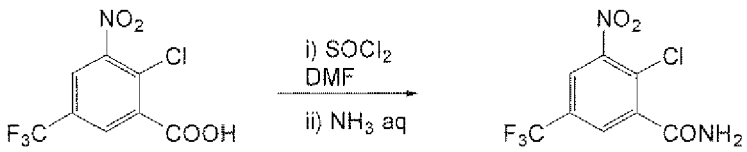


A mixture of 2-chloro-3-nitro-5-trifluoromethylbenzoic acid (1.0015g, 3.7 mmol), chloroform (5 mL), DMF (375 $\mu$L) and thionyl chloride (630 $\mu$L, 8.6 mmol) was refluxed under $N_2$ for 3 hr. The mixture was cooled to ambient temperature and evaporated to dryness (<30°C). The residue was slurried in acetonitrile (2 mL) and ice cold ammonium hydroxide solution (12 mL) was added *(vigorous reaction!)*. The mixture was stirred for 30 min at 0°C then the precipitate was isolated by vacuum filtration, and washed with cold water. The product was dried overnight *in vacuo* to give the desired product as a white solid 0.8206 g (82.1 %). LCMS: $R_t$ = 5.60 min, 100 A% @ 254 nm, $[M+H]^+$ = 269.0. $^1$H NMR (400 MHz, $CDCl_3$) $\delta$: 8.03 (dq, 1H, *J* = 0.6, 2.2), 7.88 (dq, 1H, *J* = 0.7, 2.2), 7.84 (br s, 1H), 7.29 (br s, 1H).

Preparation of 2-(methylthio)-8-nitro-6-(trifluoromethyl-4H-benzo[e][1,3]thiazin-4-one (BTZ-SMe)

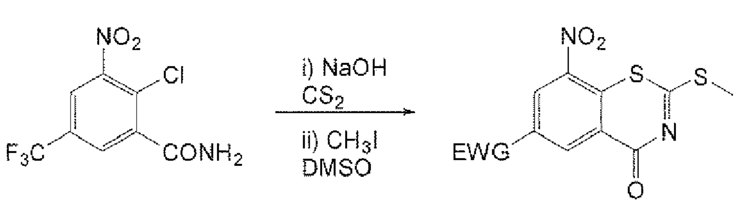


**BTZ-SMe**

Powdered sodium hydroxide (0.234 g, 5.85 mmol) was added to a solution of 2-chloro-3-nitro-5-(trifluoromethyl)benzamide (0.800 g, 3.0 mmol) in DMSO (2.8 mL). Immediately, carbon disulfide (0.55 mL, 8.9 mmol) is slowly added to the solution at <10°C. After 15 min iodomethane (185 $\mu$L, 3.0 mmol) is slowly added and formation of a solid is observed. The reaction mixture is stirred for further 35 min and then water (18 mL) is added at <20°C. The precipitated yellow solid is isolated by vacuum filtration (0.1950 g). LCMS: $R_t$ = 7.03 min, 82.6 A% @ 254 nm. The crude product

was stirred out from ethyl acetate (2.09 g) to give BTZ-SMe as pale lemon crystals, yield = 63%. LCMS: $R_t$ = 7.04 min, 99.3 A% @ 254 nm, $[M+H]^+$ = 323.0. $^1H$ NMR (400 MHz, $d_6$-DMSO) δ: 8.96 (br s, 1H), 8.83 (br s, 1H), 2.76 (s, 3H).

Reaction of the thiomethyl intermediate (II) with an amine (III) In the following a general procedure for reaction of a thiomethyl intermediate of formula (II) with an amine of formula (III) is described for obtaining the compounds of formula (I) according to the present invention.

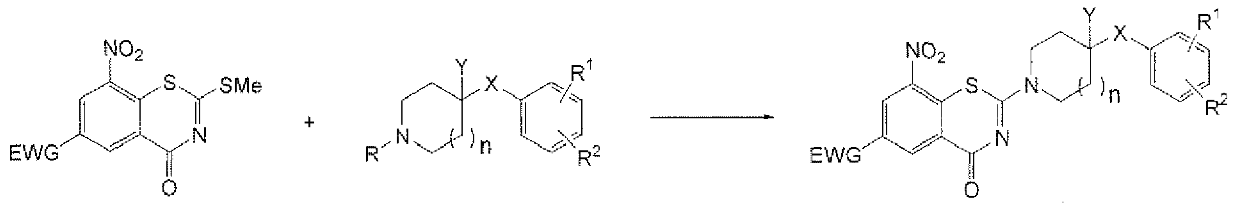

(II) (III) (I)

A mixture of a thiomethyl intermediate of formula (II) (1.0 eq), an amine of formula (III) (at least 1.2 eq) and ethanol (or THF) (20 vol) is heated to 60°C. If the amine of formula (III) was supplied as the salt (e.g. as the hydrochloride salt), 1.0 - 1.5 eq of DIPEA per equivalent of acid, is included in the reaction mixture. When analysis (TLC or HPLC) shows that the reaction is complete, the reaction is cooled to ambient temperature, the solvents are removed and the product according to formula (I) is purified.

Synthesis of 2-(4-phenylpiperidin-1-yl)-8-nitro-6-(trifluoromethyl)-4H-benzo[e][1,3]thiazin-4-one

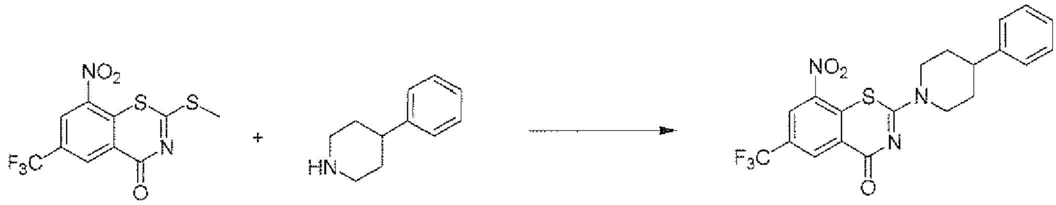

BTZ-SMe 4-phenylpiperidine MCC_000040.001

2-(4-Phenylpiperidin-1-yl)-8-nitro-6-(trifluoro-methyl)-4H-benzo[e][1,3]thiazin-4-one (MCC_00040.001) was synthesized in line with the general procedure outlined above by reacting the thiomethyl intermediate (BTZ-SMe) with the respective amine (4-phenylpiperidine), as shown above. The product was purified by flash chromatography over silica gel (45×10 mm, gradient elution 85:15 to 75:25 to 66:33 hexanes:acetone) $R_f$ = 0.43 @ 66:33 hexanes:acetone. Isolated 19.6 mg (70.4%). LCMS (Xterra MS): $R_t$ = 7.93 min, 97.6 A% @ 254 nm, $[M+H]^+$ = 436.0. $^1H$ NMR (400 MHz, $CDCl_3$) δ: 9.12 (dq, 1H, J = 0.6, 2.2), 8.76 (dq, 1H, J = 0.6, 2.2), 7.36 7.20 (m, 5H), 5.46, 4.55, 3.38, 3.12 (4× br s, 4×1H), 2.94 (tt, 1H, J = 3.7, 12.1), 2.13, 2.09 (br d, 2H, J = 12.2), 1.82 (dq, 2H, J = 4.1, 12.9).

Synthesis of 2-(4-(4-chlorophenyl)piperidin-1-yl)-8-nitro-6-(trifluoromethyl)-4H-benzo[e][1,3]thiazin-4-one

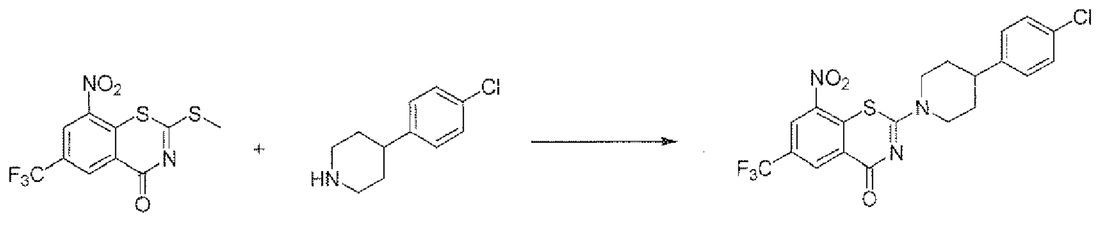

BTZ-SMe

4-(4-chlorophenyl)piperidine

BB4659_54
MCC_000376.001

2-(4-(4-Chlorophenyl)piperidin-l-yl)-8-nitro-6-(trifluoromethyl)-4H-benzo[e][1,3]thiazin-4-one (MCC_000376.001) was synthesized by reaction of BTZ-SMe (30 mg, 0.093 mmol) and 4-(4-chlorophenyl)-piperidine (27 mg, 0.14 mmol, 1.5 eq) in THF according to the general procedure, as outlined above. The product was purified by flash chromatography over silica gel (45×10 mm, 4:1 hexanes:acetone) $R_f$ = 0.45 @ 7:3 hexanes:acetone. Isolated 42 mg (96%). LCMS (Xterra MS): $R_t$ = 5.88 min, 100 A% @ 254 nm, $[M+H]^+$ = 470.0. $^1H$ NMR, (400 MHz, $CDCl_3$) δ: 9.12 (dq, 1H, J = 0.5, 2.0), 8.77 (dq, 1H, J = 0.5, 2.1), 7.29 (m, 2H), 7.14 (m, 2H), ~5.5 (b, 1H), ~4.6 (b, 1H), ~3.2 (b, 2H), 2.93 (ddt, $^1H$, J = 3.5, 8.5, 12.3), 2.09 (bd, 2H, J = 12.0), 1.79 (ddd, 2H, 3.8, 12.9).

Synthesis of 8-nitro-2-(4-(p-tolyl)piperidin-1-yl)-6-(trifluoromethyl)-4H-benzo[e][1,3]-thiazin-4-one

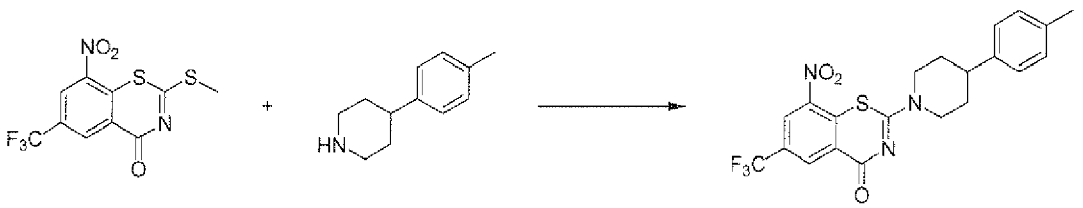

BTZ-SMe

4-(*p*-tolyl)piperidine

BB4659_55
MCC_000377.001

8-Nitro-2-(4-(p-tolyl)piperidin-yl-yl)-6-(trifluoro-methyl)-4H-benzo[e][1,3]-thiazin-4-one (MCC_000377.001) was synthesized by reaction of BTZ-SMe (30 mg, 0.093 mmol) and 4-(p-tolyl)piperidine (24 mg, 0.14 mmol, 1.5 eq) in THF according to the general procedure, as outlined above. The product was purified by flash chromatography over silica gel (45×10 mm, 4:1 hexanes:acetone) $R_f$ = 0.41 @ 7:3 hexanes:acetone. Isolated 26 mg (62%). LCMS (Xterra MS): $R_t$ = 5.75 min, 46.3 A% @ 254 nm, $[M+H]^+$ = 450.0. $^1H$ NMR (400 MHz, $CDCl_3$) δ: 9.12 (dq, 1H, J = 0.5), 8.77 (dq, 1H, J = 0.5), 7.12 (m, 4H), ~5.5 (b, 1H), ~4.5 (b, 1H), ~3.2 (b, 2H), 2.92 (m, 1H), 2.33 (s, 3H), 2.09 (bd, 2H, J ~ 8.8), 1.81 (m, 2H); $^{13}C$ NMR (400 MHz, $CDCl_3$) δ: 166.6, 161.7, 140.8, 136.5, 133.3, 129.4, 126.5, 126.0, 47.4, 42.1, 33.2, 21.0.

Synthesis of 2-(4-(3-fluorophenyl)piperidin-1-yl)-8-nitro-6-(trifluoromethyl)-4H-benzo[e][1,3]thiazin-4-one

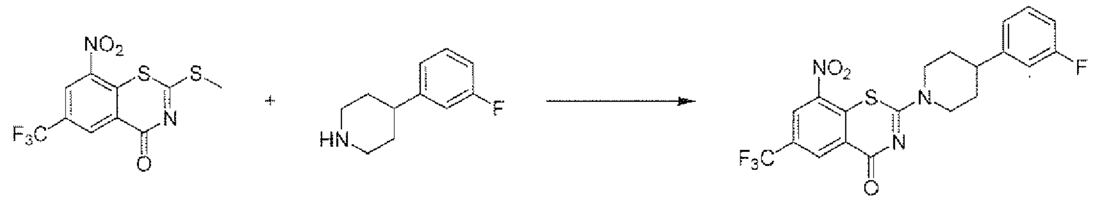

BTZ-SMe    4-(3-fluorophenyl)piperidine    BB4659_56 MCC_000378.001

2-(4-(3-Fluorophenyl)piperidin-1-yl)-8-nitro-6-(trifluoromethyl)-4H-benzo[e][1,3]thiazin-4-one (MCC_000378.001) was synthesized by reaction of BTZ-SMe (30 mg, 0.093 mmol) and 4-(3-fluorophenyl) piperidine (25 mg, 0.14 mmol, 1.5 eq) in THF according to the general procedure, as outlined above. The product was purified by flash chromatography over silica gel (45×10 mm, 4:1 hexanes:acetone) $R_f$ = 0.34 @ 7:3 hexanes:acetone. Isolated 42mg (99%). LCMS (Xterra MS): $R_t$ = 5.63 min, 69.8 A% @ 254 nm, $[M+H]^+$ = 454.0. $^1$H NMR (400 MHz, $CDCl_3$) δ: 9.11 (dq, 1H, J = 2.0), 8.77 (dq, 1H, J = 2.3), 7.29 (m, 1H), 6.98 (m, 1H), 6.92 (m, 1H), ~5.5 (b, 1H), ~4.5 (b, 1H), ~3.2 (b, 2H), 2.96 (ddt, 1H, J = 3.8, 8.5, 12.0), 2.11 (bd, 2H, J ~ 9.6), 1.88 (ddd, 2H, J = 4.1, 8.5, 12.3), 1.67 (m, 1H); $^{13}$C NMR (400 MHz, $CDCl_3$) δ: 166.5, 164.2, 161.8, 146.4, 133.3, 130.2, 126.7, 126.0, 122.3, 113.5, 47.1, 42.2, 32.9.

Synthesis of 2-(4-(3,4-difluorophenyl)piperidin-1-yl)-8-nitro-6-(trifluoromethyl)-4H-benzo[e][1,3]thiazin-4-one

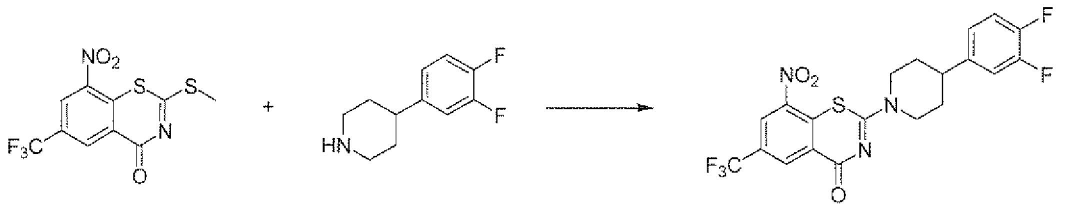

BTZ-SMe    4-(3,4-difluorophenyl)piperidine    TK4967-041D MCC_000443.001

2-(4-(3,4-Difluorophenyl)piperidin-l-yl)-8-nitro-6-(trifluoromethyl)-4H-benzo[e][1,3]thiazin-4-one (MCC_000443.001) was synthesized by reaction of BTZ-SMe (20 mg, 62.1 μmol) and 4-(3,4-difluorophenyl)piperidine (12 mg, 62.1 μmol) in ethanol according to the general procedure, as outlined above. The product was purified by flash chromatography over silica gel (150×10 mm, 1:99 to 2:98 methanol:dichloromethane) Isolated 10 mg (34%). LCMS (PhHx): $R_t$ = 9.15 min, >99 A% @ 254 nm, $[M+H]^+$ = ESI-MS 472.0 $[M+H]^+$. $^1$H NMR (400 MHz, $CDCl_3$) δ: 9.11 (dq, 1H, J = 2.1, 0.7), 8.77 (dq, 1H, J = 0.7, 2.1), 7.12 (dt, 1H, J = 10.2, 8.4), 7.01 (ddd, 1H, J = 2.1, 7.5, 11.5), 6.93 (m, 1H, J = 1.6, 3.7, 7.9), 5.44 (br s, 1H), 4.59 (br s, 1H), 3.23 (br s, 2H), 2.92 (tt, 1H, J = 12.1, 3.6), 2.10 (br d, 2H, J = 13.1), 1.76 (br dq, 2H, J = 13.1, 4.0); $^{13}$C NMR (100 MHz from gHSQC correlated protons in brackets, $CDCl_3$) δ: 133.4 (9.11, p), 126.2 (8.77, p), 122.5 (6.93, p), 117.5 (7.12, p), 115.5 (7.01, p), 41.7 (2.92, p), 33.0 (2.10, n + 1.76, n). $^{19}$F NMR (376 MHz, $CDCl_3$) δ: 140.28 (ddd, 1H, J = 4.3, 7.5, 11.5, 21.1), 137.15 (dddd, 1H, J = 1.3, 8.3, 11.3, 21.1), 62.99 (s, 3H).

Synthesis of 2-(4-hydroxy-4-phenylpiperidin-1-yl)-8-nitro-6-(trifluoromethyl)-4H-benzo[e][1,3]thiazin-4-one

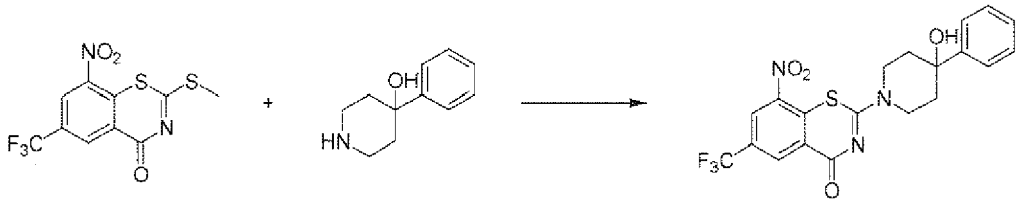

BTZ-SMe 4-phenylpiperidin-4-ol TSH5061-007D MCC_000462.001

2-(4-Hydroxy-4-phenylpiperidin-1-yl)-8-nitro-6-(trifluoromethyl)-4H-benzo[e][1,3]thiazin-4-one (MCC_000462.001) was synthesized by reaction of BTZ-SMe (30 mg, 93 μmol) and 4-phenylpiperidin-4-ol (19.8 mg, 111 μmol) in ethanol according to the general procedure, as outlined above. The product was purified by flash chromatography over silica gel (45×10 mm, 98:2 dichloromethane: methanol) $R_f$ = 0.20 @ 98:2 dichloromethane:methanol. Isolated 2.0 mg (8%). LCMS (PhHx): $R_t$ = 5.08 min, 84 A% @ 254 nm, $[M+H]^+$ = 452.0. $^1H$ NMR (400 MHz, $CDCl_3$) δ: 8.95 (dq, 1H, J = 2.0), 8.73 (dq, 1H, J = 2.0), 7.40 (m, 2H), 7.26 (m, 2H), 7.17 (m, 1H), ~5.1 (b, 1H), ~4.2 (b, 1H), ~3.3 (b, 1H). NMR shows larger amount of an impurity of unknown origin that obscures some signals.

Synthesis of 2-(4-benzylpiperidin-1-yl)-8-nitro-6-(trifluoromethyl)-4H-benzo[e][1,3]thiazin-4-one

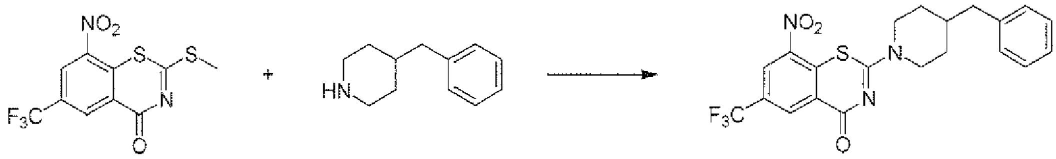

BTZ-SMe 4-benzylpiperidine TSH5061-009C MCC_000464.001

2-(4-Benzylpiperidin-1-yl)-8-nitro-6-(trifluoro-methyl)-4H-benzo[e][1,3]thiazin-4-one (MCC_000464.001) was synthesized by reaction of BTZ-SMe (30 mg, 93 μmol) and 4-benzylpiperidine (19.9 μL, 111 μmol) in ethanol according to the general procedure, as described above. The product was purified by flash chromatography over silica gel (45×10 mm, gradient 99:1 to 97:3 dichloromethane:methanol) $R_f$ = 0.63 @ 98:2 dichloromethane: methanol. Isolated 4.2mg (10%). LCMS (PhHx): $R_t$ = 9.54 min, 95 A% @ 254 nm, $[M+H]^+$ = 450.1. $^1H$ NMR (400 MHz, $CDCl_3$) δ: 9.10 (dq, 1H, J = 2.1), 8.74 (dq, 1H, J = 2.1), 7.20 (m, 2H), 7.23 (m, 1H), 7.14 (m, 2H), ~5.3 (b, 1H), ~4.4 (b, 1H), ~3.2 (b, 1H), ~2.9 (b, 3H), 2.60 (d, 2H, J = 6.7), 1.96 (m, 1H), 1.90 (m, 2H), 1.36 (dq, 2H, J = 13.0, 4.1).

Synthesis of 8-nitro-2-(4-(m-tolyl)piperidin-1-yl)-6-(trifluoromethyl)-4H-benzo[e][1,3]thiazin-4-one

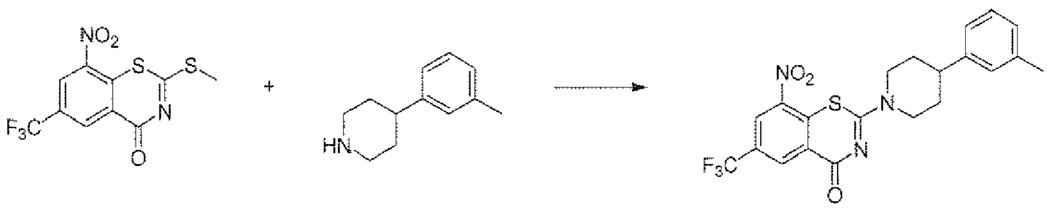

BTZ-SMe 4-(*m*-tolyl)piperidine TSH5061-018B MCC_000465.001

8-Nitro-2-(4-(m-tolyl)piperidin-1-yl)-6-(trifluoromethyl)-4H-benzo[e] [1,3]thiazin-4-one (MCC_000465.001) was synthesized by reaction of BTZ-SMe (20 mg, 62.1 μmol) and 4-(m-tolyl)piperidine (11.4 mg, 65 μmol) in THF according to the general procedure, as described above. The product was purified by flash chromatography over silica gel (45×10 mm, gradient 99:1 to 97:3 dichloromethane: methanol) $R_f$ = 0.84 @ 98:2 dichloromethane:methanol. Isolated 1.9 mg (7%). LCMS (PhHx): $R_t$ = 5.78 min, 85 A% @ 254 nm, $[M+H]^+$= 450.1. $^1H$ NMR (400 MHz, $CDCl_3$) δ: 9.12 (dq, 1H, J = 2.1), 8.76 (dq, 1H, J = 2.1), 7.22 (t, 1H, J = 7.6), 7.03 (m, 3H) ~5.4 (b, 1H), ~4.6 (b, 1H), ~3.3 (b, 2H), 2.90 (tt, 1H, J = 12.0, 3.5), 2.54 (s, 3H), ~2.1 (b, 2H), 1.82 (dq, 2H, J = 13.0, 4.4).

Synthesis of 2-(4-(3,5-difluorophenyl)piperidin-1-yl)-8-nitro-6-(trifluoromethyl)-4H-benzo[e] [1,3] thiazin-4-one

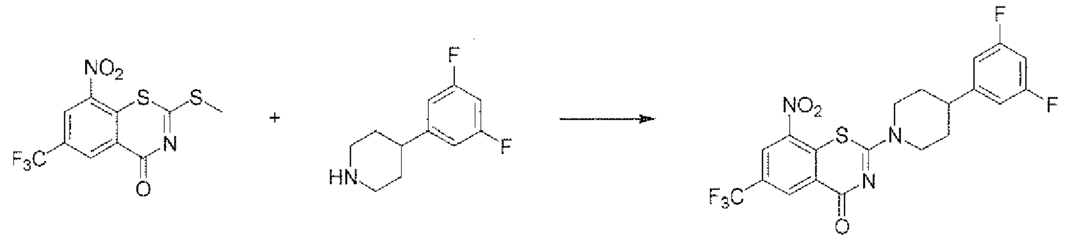

BTZ-SMe 4-(3,5-difluorophenyl)piperidine TSH5061-017B MCC_000466.001

2-(4-(3,5-Difluorophenyl)piperidin-1-yl-)-8-nitro-6-(trifluoromethyl)-4H-benzo [e] [1,3]thiazin-4-one (MCC_000466.001) was synthesized by reaction of BTZ-SMe (20 mg, 62.1 μmol) and 4-(3,5-difluorophenyl)piperidine (13.0 mg, 66 μmol) in THF according to the general procedure, as described above. The product was purified by flash chromatography over silica gel (45×10 mm, gradient 99:1 to 97:3 dichloromethane:methanol) $R_f$ = 0.90 @ 98:2 dichloro-methane:methanol. Isolated 1.9mg (7%). LCMS (PhHx): $R_t$ = 5.69 min, 87 A% @ 254 nm, $[M+H]^+$ = 472.0. $^1H$ NMR (400 MHz, $CDCl_3$) δ: 9.12 (dq, 1H, J = 2.3), 8.78 (dq, 1H, J = 2.3), 6.71 (m, 3H), ~5.5 (b, 1H), ~4.6 (b, 1H), ~3.2 (b, 2H), 2.95 (tt, 1H, J = 12.0, 4.0), 2.1 (bd, 2H), 1.77 (dq, 2H, J = 13.0, 4.0).

Synthesis of 2-(4-(2-fluorophenyl)piperidin-1-yl)-8-nitro-6-(trifluoromethyl)-4H-benzo[e] [1,3]thiazin-4-one

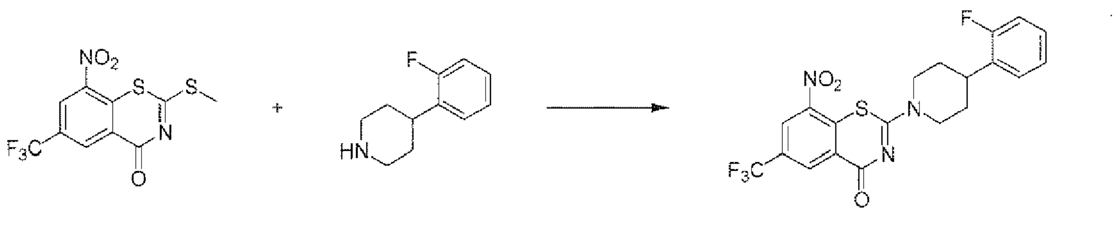

BTZ-SMe 4-(2-fluorophenyl)piperidine TK4967-014B MCC_000469.001

2-(4-(2-Fluorophenyl)piperidin-1-yl)-8-nitro-6-(trifluoromethyl)-4H-benzo[e] [1,3]thiazin-4-one (MCC_000469.001) was synthesized by reaction of BTZ-SMe (20 mg, 62.1 μmol) and 4-(2-fluorophenyl)-piperidine (12.2 mg, 61.7 μmol) in THF according to the general procedure, as described above. The product was purified by preparative HPLC to yield 10 mg (34%). LCMS (PhHx): $R_t$ = 9.14 min, >99.9 A% @ 254 nm, $[M+H]^+$ = ESI-MS 454.0. $^1H$ NMR (400 MHz, $CDCl_3/CD_3OD$) δ: 9.06 (dq, 1H, J = 2.2, 0.7), 8.84 (dq, 1H, J = 2.1, 0.7), 7. 27 7.20 (m, 2H), 7.15 7.03 (m, 2H), 5.44 (br s, 1H), 4.57 (br s, 1H), 3.53 (br s, 1H), 3.34 (tt, 1H, J = 12.2, 3.6), 3.15 (br s, 1H), 2.12 (br s, 2H), 1.90 (br dq, 2H, J = 3.4, 12.7). $19_F$ NMR (376 MHz, $CDCl_3/CD_3OD$) δ: 119.99 (ddd, 1H, J = 5.5, 7.5, 12.5), 77.07 (s, 1H, TFAOH), 64.02 (s, 3H). HR-ESI-MS calc. for $C_{20}H_{16}F_4N_3O_3S$: 454.0843, found: 454.0850.

**Biological examples**

The compounds of the present invention can be tested by various methods and generally show a high activity

against *Mycobacteria,* i.e. a low minimum inhibitory concentration (MIC) in the nanomolar range.

**Methods**

MIC Assay Method

The protocol used is the broth microdilution method in microtiter plates (MTP), based upon that detailed by the CLSI (Clinical and Laboratory Standards Institute (CLSI). 2006. Methods for Dilution Antimicrobial Susceptibility Tests for Bacteria that Grow Aerobically; Approved Standard. 7th edn. Document M7-A6. Clinical and Laboratory Standards Institute, Wayne, PA.).

Strain: *Mycobacterium smegmatis,* ATCC 700084, or other *Mycobacterium* strains (see table below).

Medium: The bacteria culture and the MIC were done using the following medium: **MHB** - Mueller-Hinton Broth + 0.05% Tween80, purchased from Difco, Tween80 has been added to avoid clumping of the bacteria.

Preparation of compound solutions: The compounds are dissolved in DSMO in a concentration of 1 mg/ml and further diluted in water to achieve the final concentration, which is used as the highest concentration for determination of MIC. This highest concentration is filled in the first row of the microtiter plate and further diluted in the culture medium for the *Mycobacteria* (Mueller-Hinton Broth and Middlebrook 7H9 broth). All solutions are freshly prepared under sterile conditions.

Resazurin salt powder (Sigma) is prepared at 0.01 % (wt/vol) in distilled water, sterilized by filtration through a 0.22 μm membrane and stored at 4°C for a week.

Ciprofloxacin, isoniaz-d and rifampicin can be used as reference compounds for control of the assay as well as a solvent control. Minimal inhibitory concentrations (MIC) are determined by the broth microdilution method. 50 μl of the compound solution in DMSO/MeOH are serially diluted directly in the microtiter plate by factor two with Mueller-Hinton broth (MHB)(Difco). Then the wells are inoculated with 50 μl of test organisms in MHB to give a final concentration of $5x10^5$ CFU/ml. After microtiter plates are incubated at 37°C for 48 h, the optical density of each well is measured with a Nepheloscan Ascent 1.4 automatic plate reader (Labsystems, Vantaa, Finland). Then 30 μl of resazurin solution is added to each well and plates are allowed to incubate at 37°C for additional 24 h. A change from blue to pink indicates reduction of resazurin and therefore bacterial growth. The MIC is defined as the lowest drug concentration that prevents this colour change.

Agar Diffusion Assay Method

Antimicrobial activity is additionally determined by agar diffusion tests according to: European Pharmacopoeia, 3rd edition, 1997; 13, 118, Deutscher Apotheker Verlag Stuttgart

100 μl suspension of the test organism *M.* smegmatis *SG987* with a density of McFarland standard 0.5 are inoculated into 34 ml of sterile melted agar medium (*Nutrient agar* from Serva Electrophoresis GmbH, Heidelberg, Germany) and poured into Petri dishes. Wells of 9 mm in diameter are cut in the agar and filled with 50 μl of the compound solution (for concentrations see Table 2). Compounds are dissolved in DMSO in a concentration of 1 mg/ml and further diluted in methanol to the final concentration. Growth inhibition zones around the agar wells are read after overnight incubation at 37°C.

**Bacterial strains, growth conditions and plasmids**

*M. tuberculosis* H37Rv is used as the reference strain. The recombinant strain of *M. tuberculosis* H37Rv expressing the green fluorescent protein (H37Rv-GFP) bears an integrative plasmid (based on Ms6) carrying a *gfp* gene constitutively expressed from the promoter *pBlaF*. Both strains are grown at 37°C in Middlebrook 7H9 broth (Difco) supplemented with 0.05% Tween 80 and albumindextrose-catalase (ADC) or on solid Middlebrook 7H11 medium (Difco) supplemented with oleic acid-albumindextrose-catalase (OADC). When required, the media are supplemented with the following concentrations of antibiotics: 20 μg/ml of kanamycin, 50 μg/ml of hygromycin. To assess BTZ efficacy on non-growing cells, the streptomycin dependent model (our unpublished work) and the nutrient starvation model are used. Drug susceptibility to clinical isolates, including MDR-and XDR-strains, is performed at the Central Institute for Tuberculosis, Moscow and at the National Reference Center for *Mycobacteria,* Borstel.

**BTZ efficacy in the chronic model**

Animal efficacy can be determined in a standard mouse infection model according to e.g. the following protocol. BALB/c mice are infected with a low bacillary load (~200 CFU) of M. tuberculosis H37Rv via aerosol. Treatment starts four-weeks post infection. Mice are dosed by gavage with 37.5, or 300 mg of BTZ, per kg body weight, in carboxymethyl

cellulose formulation (0.25%), once daily, six times/week, for four weeks. Control and treated mice are sacrificed, lungs and spleens homogenized and dilutions plated for enumeration of viable bacilli.

**Results**

The obtained results are summarized in the following Table 1 and Table 2:

Table 1:

| | Compound | MIC [ng/mL] | Chemical Stability[1] [%] | Metabolic Stability[2] [%] | Plasma Stability[3] [%] | In-vivo Mouse 50 img/kg |
|---|---|---|---|---|---|---|
| | | *M. smeg. SG987*<br>*M. vaccae 10670*<br>*M fort.*<br>*M. aurum*<br>*M. 1b.* | | Human<br>Mouse | Human<br>Mouse | Survival<br>4 wks |
| **1** | 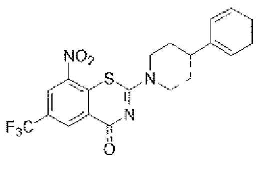<br>MCC_000040.001 | 4<br>0.4<br>12.5<br>>100<br><15 | 82 | 120<br>75 | 96<br>71 | 4 of 10 |
| **2** | 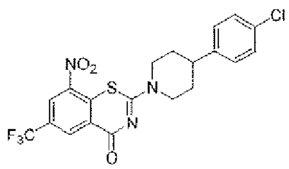<br>MCC_000376.001 | <5<br><0.0012<br>11. t.<br>n. t.<br>4 | 52 | 59<br>39 | 86<br>88 | n. t. |
| 3 | 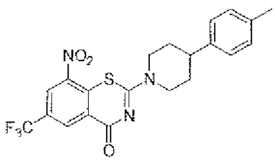<br>MCC_000377 001 | <5<br><0.0012<br>n.t.<br>n. t.<br>8 | 54 | 30<br>9 | 98<br>108 | n.t. |
| 4 | 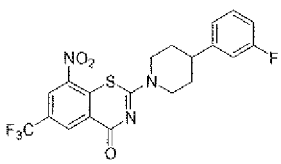<br>MCC_000378.001 | <5<br><0.0012<br>n. t.<br>n. t.<br>16 | 71 | 28<br>10 | 99<br>107 | n. t. |
| 5 | 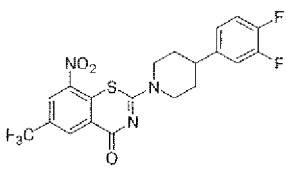<br>MCC_000443.001 | <5<br><0.0012<br>n. t.<br>n.t.<br>8 | 63 | 49<br>28 | 124<br>111 | n. t. |

(continued)

| | Compound | MIC [ng/mL] | Chemical Stability[1] [%] | Metabolic Stability[2] [%] | Plasma Stability[3] [%] | In-vivo Mouse 50 img/kg |
|---|---|---|---|---|---|---|
| 6 | 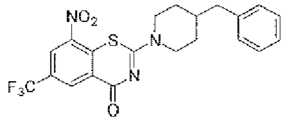<br>MCC_000464 001 | <5<br><0.0012<br>n. t.<br>n. t.<br>32 | 55 | 1<br>0 | 98<br>108 | n. t. |
| 7 | 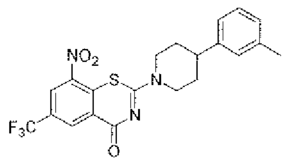<br>MCC_000465.001 | <5<br><0.0012<br>n. t.<br>n. t.<br>8 | 53 | 21<br>18 | 103<br>100 | n. t. |
| **8** | 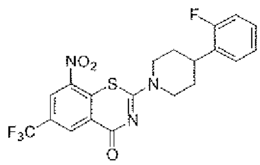<br>MCC_000469.001 | <5<br><0.0012<br>n. t.<br>n. t.<br>8 | 61 | 69<br>78 | 107<br>105 | n.t. |
| **I (Ref.)** | 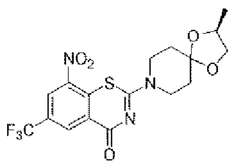<br>BTZ_10526043.003<br>(Compound 1 of WO 2007/134625) | <5<br>0.625<br>1.56<br>>100000<br><15 | 98 | 107<br>97 | 98<br>45 | n. t. |
| **II (Ref.)** | 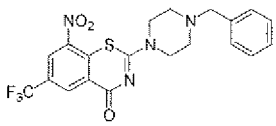<br>(Compound 7 of WO 2009/010163) | 50<br>50<br>50<br>n. t.<br>n. t. | n. t. | n. t. | n. t. | n. t. |
| **III (Ref.)** | 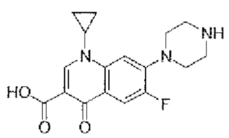<br>(Ciprofloxacin) | 200<br>100<br>n. t.<br>n. t.<br>n.t. | n. t. | n. t. | n. t. | n. t. |

n. .t = not tested.
[1] Test compound 5 μM in 35mM NaCl, 85 mM HCl, appr. 2000 U pepsin.
[2] Test compound 1 μM in NADP (1.3 mM). G6P (D-Glucose-6-phosphate, 3.3 mM), G6PDHase (Glucose-6-phosphate dehydrogenase. 0.,4 U/ml), 0,01% DMSO (see Kuhnz. W. and Gieschen, H., Drug Metab Dispos 1998. 26. p. 1120-1127).
[3] Test compound 5 μM in plasma (see Singh et al., Rapid Commun. Mass Spectrom 1996, 10, p. 1019-1026).

Table 2: Data of agar diffusion assay (*M. smegmatis SG987*).

| | Compound | Inhibition zone at 1 μg/ml [mm] |
|---|---|---|
| **2** | 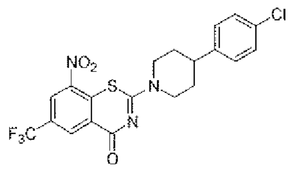 MCC_000376.001 | 22 |
| **3** | 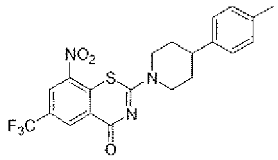 MCC_000377.001 | <22 |
| **4** | 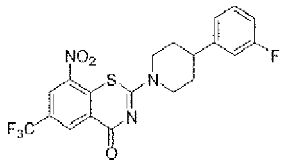 MCC_000378.001 | 24 |
| **5** | 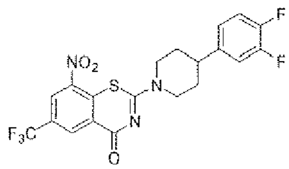 MCC_000443.001 | 27 |
| **8** | 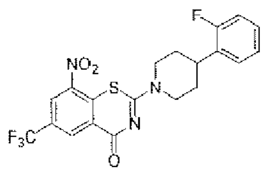 MCC_000469.001 | 27 |
| **I (Ref.)** | 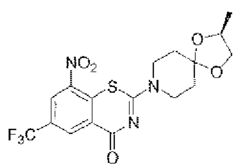 BTZ_10526043.003 (Compound 1 of WO 2007/134625) | 17 p-P4 |

(continued)

| | Compound | Inhibition zone at 1 μg/ml [mm] |
|---|---|---|
| **III (Ref.)** | 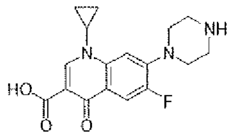 (Ciprofloxacin) | 20p[4] |

[4]p)- p - P - A: indication for increasing numbers of colonies in the inhibition zone (p) = few colonies in the inhibition zone, p = colonies in the inhibition zone, P = many colonies in the inhibition zone, A = faint indication of inhibition zone).

Discussion

The minimal inhibitory concentrations (MIC) of a variety of compounds of formula (I) against different *Mycobacteria* are very low, and generally in the range of 0.001-20 ng/ml for most *Mycobacteria* strains tested. As shown above, the MIC for one of the most preferred compounds of the present invention, Compound 2, against MDR *M. tuberculosis* is 4 ng/ml, against M. *smegmatis SG987* <5 ng/ml and against M. vaccae *10670* <0.0012 ng/ml, respectively (see Table 1), which compares favorably with those of the existing anti-tuberculosis drugs, isoniazid, INCH, (0.02-0.2 μg/ml) and ethambutol, EMB, (1-5 μg/ml). Moreover, the MIC of Compound 2 of the present invention is at least equal to the activity of compound 1 of WO 2007/134625 (Table 1, Ref I).

Additionally, antimicrobial activity of the compounds according to the present invention was tested in the agar diffusion assay against *Mycobacterium smegmatis SG987* as test organism. Table 2 shows the determined inhibition zones, e.g. 27 mm for Compound 8 as one of the most preferred compounds of the present invention. In contrast, ciprofloxacin as known drug is characterized by an inhibition zone of only 20p mm (p denotes an increase of colony numbers in the inhibition zone).

The compounds of the present invention, and particularly Compound 2 and Compound 8, appear to be suitable candidates for development into a sterilizing anti-tuberculosis (TB) drug. It is currently assumed that the compounds of formula (I) act on the enzyme decaprenylphosporyl-β-D-ribose-2'-epimerase, but the present invention is not bound by this theory.

In addition to their excellent antibacterial activity *in vitro* and in experimental models, the compounds of the present invention unexpectedly also proved to have a favourable profile of good plasma stability and lower cytotoxicity (tested with TA98, TA100, and TA1535 strains).

The compounds of the present invention can therefore be expected to be useful in the treatment of tuberculosis and other diseases caused by *Mycobacteria,* particularly by *M. tuberculosis* and *M. smegmatis.* The invention therefore also pertains to the use of the inventive compounds and their pharmaceutically acceptable salts or solvates in the treatment of such diseases, particularly tuberculosis, and most particularly tuberculosis caused by multidrug-resistant strains of *Mycobacteria.*

## Claims

**1.** A compound of formula (I) or a pharmaceutically acceptable salt or solvate thereof,

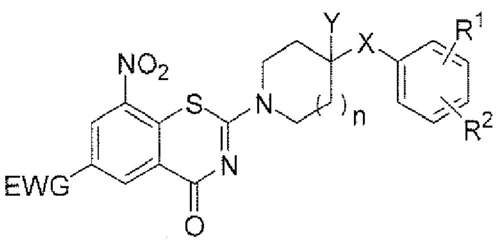

(I)

wherein

EWG (electron withdrawing group) = $NO_2$, CN, $CF_3$, F, Cl, Br, $OCF_3$, OH, OR, $OCHF_2$, COOR, wherein R is hydrogen or a straight or branched $C_1$-$C_4$ alkyl group,
X = a bond or a straight or branched $C_1$-$C_4$ alkylene group which may be substituted with a group selected from F, Cl, Br, I or $C_1$-$C_4$ alkoxy;
Y = hydrogen, a straight or branched $C_1$-$C_4$ alkyl group, OH or OR, wherein R is hydrogen or a straight or branched $C_1$-$C_4$ alkyl group;
n = 0, 1 or 2;
$R^1$, $R^2$ = hydrogen or substituent(s) which may be the same or different from each other and are selected from the group consisting of D, F, Cl, Br, $CF_3$, a straight or branched $C_1$-$C_4$ alkyl group, a phenyl group, OR, SR, $NR^3R,^4$, wherein R, $R^3$, $R^4$ may be the same or different from each other and are hydrogen or a straight or branched $C_1$-$C_4$ alkyl group or a phenyl group.

**2.** A compound of formula (I) or a pharmaceutically acceptable salt or solvate thereof according to claim 1, wherein X is a bond.

**3.** A compound of formula (I) or a pharmaceutically acceptable salt or solvate thereof according to claim 1 or 2, wherein
EWG = $CF_3$;
X = a bond;
Y = hydrogen, a straight or branched $C_1$-$C_4$ alkyl group, OH or OR, wherein R is hydrogen or a straight or branched $C_1$-$C_4$ alkyl group;
n = 1;
$R^1$, $R^2$ = hydrogen or substituent(s) which may be the same or different from each other and are selected from the group consisting of D, F, Cl, Br, $CF_3$, a straight or branched $C_1$-$C_4$ alkyl group, a phenyl group, OR, SR, $NR^3R^4$, wherein R, $R^3$, $R^4$ may be the same or different from each other and are hydrogen, or a straight or branched $C_1$-$C_4$ alkyl group or a phenyl group.

**4.** A compound of formula (I) or a pharmaceutically acceptable salt or solvate thereof according to claim 1, wherein
EWG = $CF_3$;
X = a bond;
Y = hydrogen or hydroxy;
n = 1;
$R^1$, $R^2$ = hydrogen or substituent(s) which may be the same or different from each other and are selected from the group consisting of D, F, Cl, Br, $CF_3$, a straight or branched $C_1$-$C_4$ alkyl group, a phenyl group, OR, SR, $NR^3R^4$, wherein R, $R^3$, $R^4$ may be the same or different from each other and are hydrogen or a straight or branched $C_1$-$C_4$ alkyl group or a phenyl group.

**5.** A compound according to any one of the preceding claims or a pharmaceutically acceptable salt or solvate thereof, wherein $R^1$ is hydrogen, F, Cl or a $CH_3$-group and wherein $R^2$ is hydrogen or F.

**6.** A compound according to formula (I) or a pharmaceutically acceptable salt or solvate thereof which is selected from the group consisting of:

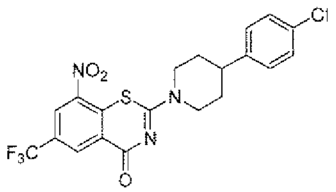

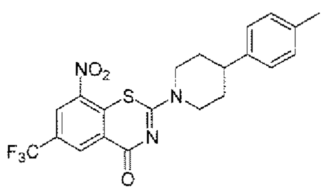

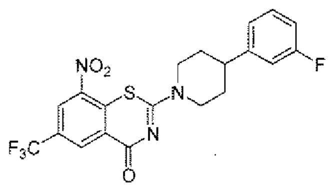

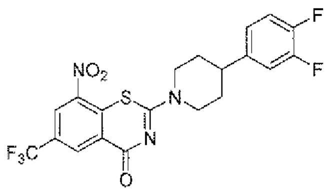

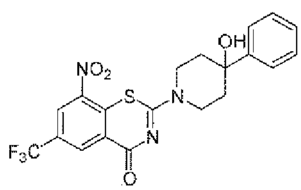

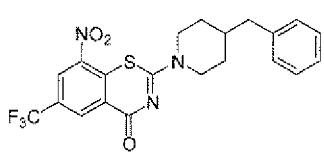

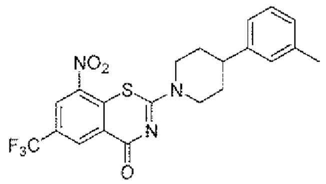

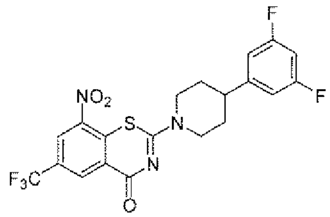

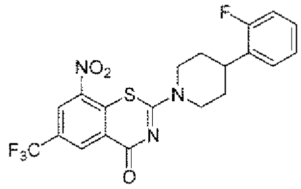

and

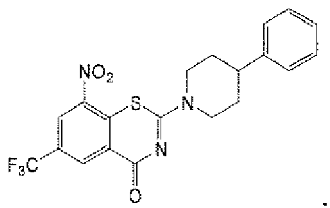

7. The compound 2-(4-(2-fluorophenyl)piperidin-1-yl)-8-nitro-6-(trifluoromethyl)-4H-benzo[e][1,3]thiazin-4-one or a pharmaceutically acceptable salt or solvate thereof.

8. The compound 2-(4-(4-chlorophenyl)piperidin-1-yl)-8-nitro-6-(tri-fluoromethyl)-4H-benzo[e][1,3]thiazin-4-one or a pharmaceutically acceptable salt or solvate thereof.

9. A compound of formula (I) or a pharmaceutically acceptable salt or solvate thereof according to any of the preceding claims for use in therapy.

10. A compound of formula (I) or a pharmaceutically acceptable salt or solvate thereof according to any of the preceding claims for use in a method for treating or preventing of a microbial infection in mammals, particularly humans.

11. The compound for use according to claim 10, wherein the microbial infection is a tuberculosis or leprosy infection.

12. A process for the preparation of a compound of formula (I) according to claim 1, comprising the steps of:

a) reacting a thiomethyl intermediate according to formula (II)

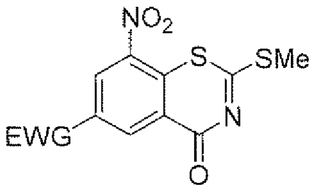

(II)

with an amine according to formula (III) or a salt thereof, wherein R is H or trimethylsilyl

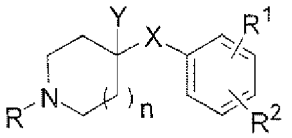

(III)

to obtain a compound of formula (I),

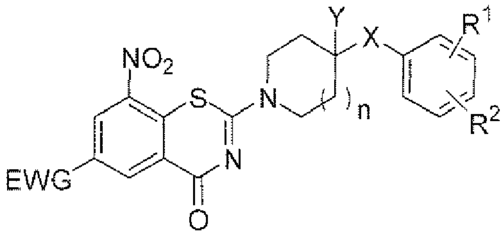

(I)

wherein
EWG (electron withdrawing group) = $NO_2$, CN, $CF_3$, F, Cl, Br, $OCF_3$, OH, OR, $OCHF_2$, COOR, wherein R is hydrogen or a straight or branched $C_1$-$C_4$ alkyl group,
X = a bond or a straight or branched $C_1$-$C_4$ alkylene group which may be substituted with a group selected from F, Cl, Br, I, or $C_1$-$C_4$ alkaxy:
Y = hydrogen, a straight or branched $C_1$-$C_4$ alkyl group, OH or OR, wherein R is hydrogen or a straight or branched $C_1$-$C_4$ alkyl group;
n = 0, 1 or 2;
$R^1$, $R^2$ = hydrogen or substituent(s) which may be the same or different from each other and are selected from the group consisting of D, F, Cl, Br, $CF_3$, a straight or branched $C_1$-$C_4$ alkyl group, a phenyl group, OR, SR, $NR^3R^4$, wherein R, $R^3$, $R^4$ may be the same or different from each other and are hydrogen or a straight or branched $C_1$-$C_4$ alkyl group or a phenyl group, and
b) optionally converting the compound of formula (I) into one of its pharmaceutically acceptable salts or solvates.

**13.** The process for the preparation of a compound of formula (I) according to claim 12, wherein the reaction is performed in THF or ethanol at 40-80°C, preferably at 55-65°C.

**14.** A pharmaceutical composition comprising a compound of formula (I) according to any of claims 1-8 or a pharmaceutically acceptable salt or solvate thereof and optionally other therapeutic agents.

**15.** A pharmaceutical composition according to claim 14 for use in a method of treating a tuberculosis infection.

Europäisches Patentamt
European Patent Office
Office européen des brevets

# EUROPEAN SEARCH REPORT

Application Number
EP 11 18 1388

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | EP 2 176 248 A1 (LEIBNIZ INST FUER NATURSTOFF F [DE]) 21 April 2010 (2010-04-21) * the whole document * ----- | 1-15 | INV. C07D417/04 A61K31/5415 A61P31/06 |
| A | AHMED KAMAL ET AL: "Anti-tubercular agents. Part 5: Synthesis and biological evaluation of benzothiadiazine 1,1-dioxide based congeners", EUROPEAN JOURNAL OF MEDICINAL CHEMISTRY, vol. 45, no. 10, 1 October 2010 (2010-10-01), pages 4545-4553, XP55010966, ISSN: 0223-5234, DOI: 10.1016/j.ejmech.2010.07.015 * the whole document * ----- | 1-15 | |

TECHNICAL FIELDS SEARCHED (IPC)

C07D

The present search report has been drawn up for all claims

1

| Place of search | Date of completion of the search | Examiner |
|---|---|---|
| Munich | 2 November 2011 | Grassi, Damian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 11 18 1388

This annex lists the patent family members relating to the patent documents cited in the above-mentioned European search report.
The members are as contained in the European Patent Office EDP file on
The European Patent Office is in no way liable for these particulars which are merely given for the purpose of information.

02-11-2011

| Patent document cited in search report | Publication date | Patent family member(s) | Publication date |
|---|---|---|---|
| EP 2176248 A1 | 21-04-2010 | AT 482939 T | 15-10-2010 |
| | | AU 2008278056 A1 | 22-01-2009 |
| | | CA 2693088 A1 | 22-01-2009 |
| | | CN 101809009 A | 18-08-2010 |
| | | CR 11240 A | 19-05-2010 |
| | | EA 201070145 A1 | 30-06-2010 |
| | | EC SP109962 A | 31-03-2010 |
| | | EP 2020406 A1 | 04-02-2009 |
| | | WO 2009010163 A1 | 22-01-2009 |
| | | JP 2010533661 A | 28-10-2010 |
| | | KR 20100043071 A | 27-04-2010 |
| | | MA 31607 B1 | 02-08-2010 |
| | | US 2010286130 A1 | 11-11-2010 |
| | | ZA 201000491 A | 27-10-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2176248 B1
- WO 2009010163 A
- WO 2007134625 A
- EP 2020406 A


### Non-patent literature cited in the description


- **ISAEW S. G.** *Farm. Zh.,* 2000, vol. 52
- **MAKOSZA M. ; NIZAMOV, S.** *Org. Prep. and Proced. Int.,* 1997, vol. 29, 707
- **NERIN C. ; TORNES A. R. ; DOMENTO C. ; CACHO J.** *J. Agr. and Food Chem.,* 1996, vol. 44, 4009
- **THIEL W. ; MAYER R. ; JAUER E.-A. ; MODROW H. ; DOST H.** *J. Prakt. Chem.,* 1986, vol. 328, 497
- **YOKOYAMA M. ; YOSHIDA S. ; IMAMOTO T.** *Synthesis,* 1982, 591
- **ROMANOWSKI J. ; ECKSTEIN Z.** *Pol. J. Chem.,* 1984, vol. 58, 263
- **NISATO D. ; SACILOTTO R. ; FRIGERIO M. ; BOVERI S. ; PALMISANO G. ; LESMA G.** *Org. Prep. Proced. Int.,* 1985, vol. 17, 75
- **OIKAWA N. ; NAKAGAWA Y. ; NISHIMURA K. ; UENO T. ; FUJITA T.** *Pestle, Sci.,* 1994, vol. 41, 139
- **WELCH D.E. ; BARON R.R.** *J. Med. Chem.,* 1969, vol. 12, 299
- **FULLER R.W. ; MOLLOY B.B. ; DAY W.A. ; ROUSH B.W. ; MARCH M.M.** *J. Med. Chem.,* 1973, vol. 16, 101
- **KUHNZ. W. ; GIESCHEN, H.** *Drug Metab Dispos,* 1998, vol. 26, 1120-1127
- **SINGH et al.** *Rapid Commun. Mass Spectrom,* 1996, vol. 10, 1019-1026